# EUROPÄISCHE PATENTANMELDUNG

(11) **EP 3 018 207 A1**
(43) Veröffentlichungstag der Anmeldung: **11.05.2016**
(21) Anmeldenummer: 15194718.1
(22) Anmeldetag: 29.01.2000
(51) Int. Cl.: C12N 15/11, A61K 31/713, A61P 43/00

(54) **OLIGORIBONUKLEOTID ZUR HEMMUNG DER EXPRESSION EINES VORGEGEBENEN GENS**

(30) Priorität: 30.01.1999 DE 19903713; 24.11.1999 DE 19956568
(62) Teilanmeldung aus: 10011217.6
(71) Anmelder: Alnylam Europe AG, 95326 Kulmbach (DE)
(72) Erfinder: KREUTZER, Roland, Cambridge, MA Massachusetts 02142 (US); LIMMER, Stefan, Cambridge, MA Massachusetts 02142 (US)
(74) Vertreter: Vossius & Partner Patentanwälte Rechtsanwälte mbB

(57) **Zusammenfassung**

Die vorliegende Erfindung umfasst zwei voneinander verschiedene 15 bis 49 Basenpaare aufweisende Oligoribonukleotide mit doppelsträngiger Struktur (dsRNAs) zur Hemmung der Expression zweier vorgegebener Zielgene in Säugerzellen, wobei ein Strang jeder dsRNA einen höchstens 49 aufeinanderfolgende Nukleotidpaare aufweisenden Bereich I aufweist, der zumindest abschnittsweise komplementär zu jeweils einem von den zwei verschiedenen Zielgenen ist, und ein innerhalb der doppelsträngigen Struktur komplementärer Bereich II jeweils aus zwei separaten RNA Einzelsträngen gebildet wird.

SEQUENZPROTOKOLL

<110> Alnylam Europe AG

<120> Oligoribonukleotid zur Hemmung der Expression eines vorgegebenen Gens

<130> N1008 EP/5

<150> 199 03 713.2
<151> 1999-01-30

<150> 199 56 568.6
<151> 1999-11-24

<160> 8

<170> PatentIn Ver. 2.1

<210> 1
<211> 45
<212> DNA
<213> Künstliche Sequenz

<220>
<223> Beschreibung der künstlichen Sequenz:
      EcoRI-Schnittstelle, T7-RNA-Polymerasepromotor

<400> 1
ggaattctaa tacgactcac tatagggcga tcagatctct agaag                45


<210> 2
<211> 50
<212> DNA
<213> Künstliche Sequenz

<220>
<223> Beschreibung der künstlichen Sequenz:
      BamHI-Schnittstelle, SP6-RNA-Polymerasepromotor

<400> 2
gggatccatt taggtgacac tatagaatac ccatgatcgc gtagtcgata           50


<210> 3
<211> 340
<212> RNA
<213> Künstliche Sequenz

<220>
<223> Beschreibung der künstlichen Sequenz: RNA, die
      einer Sequenz aus der "positive control DNA" des
      HeLaScribe Nuclear Extract in vitro
      Transkriptionskits der Firma Promega entspricht

<400> 3
ucagaucucu agaagcuuua augcgguagu uuaucacagu uaaauugcua acgcagucag 60
gcaccgugua ugaaaucuaa caaugcgcuc aucgucaucc ucggcaccgu cacccuggau 120
gcuguaggca uaggcuuggu uaugccggua cugccgggcc ucuugcggga uaucguccau 180
uccgacagca ucgccaguca cuauggcgug cugcuagcgc uauaugcguu gaugcaauuu 240
cuaugcgcac ccguucucgg agcacugucc gaccgcuuug gccgccgccc aguccugcuc 300
gcuucgcuac uuggagccac uaucgacuac gcgaucaugg                      340

<210> 4
<211> 363
<212> DNA
<213> Künstliche Sequenz

<220>
<223> Beschreibung der künstlichen Sequenz: DNA, die
      einer Sequenz aus der "positive control DNA" des
      HeLaScribe Nuclear Extract in vitro
      Transkriptionskits der Firma Promega entspricht

<400> 4
tcagatctct agaagcttta atgcggtagt ttatcacagt taaattgcta acgcagtcag 60
gcaccgtgta tgaaatctaa caatgcgctc atcgtcatcc tcggcaccgt caccctggat 120
gctgtaggca taggcttggt tatgccggta ctgccgggcc tcttgcggga tatcgtccat 180
tccgacagca tcgccagtca ctatggcgtg ctgctagcgc tatatgcgtt gatgcaattt 240
ctatgcgcac ccgttctcgg agcactgtcc gaccgctttg gccgccgccc agtcctgctc 300
gcttcgctac ttggagccac tatcgactac gcgatcatgg cgaccacacc cgtcctgtgg 360
atc                                                               363


<210> 5
<211> 315
<212> RNA
<213> Künstliche Sequenz

<220>
<223> Beschreibung der künstlichen Sequenz: Sequenz aus
      dem YFP-Gen

<400> 5
auggugagca agggcgagga gcuguucacc gggguggugc ccauccuggu cgagcuggac 60
ggcgacguaa acggccacaa guucagcgug uccggcgagg gcgagggcga ugccaccuac 120
ggcaagcuga cccugaaguu caucugcacc accggcaagc ugcccgugcc cuggcccacc 180
cucgugacca cccugaccua cggcgugcag ugcuucagcc gcuaccccga ccacaugaag 240
cagcacgacu ucuucaaguc cgccaugccc gaaggcuacg uccaggagcg caccaucuuc 300
uucaaggacg acggc                                                  315


<210> 6
<211> 52
<212> DNA
<213> Künstliche Sequenz

<220>
<223> Beschreibung der künstlichen Sequenz:
      EcoRI-Schnittstelle, T7-RNA-Polymerasepromotor,
      komplementärer Bereich zum YFP-Gen

<400> 6
ggaattctaa tacgactcac tatagggcga atggtgagca agggcgagga gc         52


<210> 7
<211> 53
<212> DNA
<213> Künstliche Sequenz

<220>
<223> Beschreibung der künstlichen Sequenz:
      BamHI-Schnittstelle, SP6-RNA-Polymerasepromotor,
      komplementärer Bereich zum YFP-Gen

<400> 7
gggatccatt taggtgacac tatagaatac gccgtcgtcc ttgaagaaga tgg          53

<210> 8
<211> 21
<212> RNA
<213> Künstliche Sequenz

<220>
<223> Beschreibung der künstlichen Sequenz: RNA, die
      einer Sequenz aus dem YFP-Gen entspricht

<400> 8
ucgagcugga cggcgacgua a                                              21

## Beschreibung

Die Erfindung betrifft Verfahren zur Hemmung der Expression eines vorgegebenen Zielgens in einer Zelle. Sie betrifft ferner ein Medikament und eine Verwendung doppelsträngiger Oligoribonukleotide.

Ein solches Verfahren ist aus der nachveröffentlichten WO 99/32619 bekannt. Das bekannte Verfahren zielt auf die Hemmung der Expression von Genen in Zellen von Invertebraten ab. Dazu ist es erforderlich, daß das doppelsträngige Oligoribonukleotid eine zum Zielgen identische Sequenz mit einer Länge von mindestens 50 Basen aufweist. Zur Erzielung einer effizienten Hemmung ist eine Länge der identischen "Sequenz von 300 bis 1000 Basenpaare erforderlich. Der Herstellungsaufwand eines solchen Oligoribonukleotids ist hoch.

Die DE 196 31 919 C2 beschreibt eine Anti-Sinn-RNA mit besonderen Sekundärstrukturen, wobei die Anti-Sinn-RNA in Form eines sie kodierenden Vektors vorliegt. Bei der Anti-Sinn-RNA handelt es sich um ein RNA-Molekül, das komplementär zu Bereichen der mRNA ist. Durch Bindung an diese Bereiche wird eine Hemmung der Genexpression bewirkt. Diese Hemmung kann insbesondere zur Diagnose und/oder Therapie von Erkrankungen, z.B. Tumorerkrankungen oder viralen Infektionen, eingesetzt werden. - Die Anti-Sinn-RNA muß nachteiligerweise in einer Menge in die Zelle eingebracht werden, die mindestens genauso groß wie die Menge der mRNA ist. Die Wirksamkeit der bekannten Anti-Sinn-Verfahren ist nicht besonders hoch.

Aus der US 5,712,257 ist ein Medikament bekannt, das fehlgepaarte doppelsträngige RNA (dsRNA) und biologisch aktive fehlgepaarte Bruchstücke von dsRNA in Form eines ternären Komplexes mit einem oberflächenaktiven Mittel enthält. Die dabei verwendete dsRNA besteht aus synthetisch hergestellten Nukleinsäureeinzelsträngen ohne definierte Basensequenz. Die Einzelstränge gehen nicht-reguläre, sogenannte "Nicht-Watson-Crick"-Basenpaarungen miteinander ein, so daß fehlgepaarte Doppelstränge gebildet werden. Die bekannte dsRNA dient zur Hemmung der Vermehrung von Retroviren, wie HIV. Die Vermehrung des Virus kann gehemmt werden, wenn nicht-sequenzspezifische dsRNA in die Zellen eingebracht wird. Es kommt dabei zu einer Induktion von Interferon, wodurch die Virusvermehrung gehemmt werden soll. Der hemmende Effekt bzw. die Wirksamkeit dieses Verfahrens ist gering.

Aus Fire, A. et.al, NATURE, Vol. 391, pp. 806 ist es bekannt, daß dsRNA, deren einer Strang abschnittsweise komplementär zu einem zu hemmenden Gen eines Fadenwurms ist, die Expression dieses Gens mit einer hohen Wirksamkeit hemmt. Es wird die Auffassung vertreten, daß die besondere Wirksamkeit der verwendeten dsRNA in Zellen des Fadenwurms nicht auf dem Anti-Sinn-Prinzip beruht, sondern möglicherweise auf katalytische Eigenschaften der dsRNA bzw. durch sie induzierte Enzyme zurückzuführen ist. - Über die Wirksamkeit spezifischer dsRNA in bezug auf die Hemmung der Genexpression, insbesondere in Säugerzellen und humanen Zellen, ist in diesem Artikel nichts ausgesagt.

Aufgabe der vorliegenden Erfindung ist es, die Nachteile nach dem Stand der Technik zu beseitigen. Es soll insbesondere ein möglichst effizientes Verfahren, Medikament bzw. eine möglichst effiziente Verwendung zur Herstellung eines Medikaments angegeben werden, mit dem/der eine besonders wirksame Hemmung der Expression eines vorgegebenen Zielgens bewirkbar ist.

Die Aufgabe wird durch die Merkmale der Ansprüche 1 bis 3 gelöst. Vorteilhafte Ausgestaltungen ergeben sich aus den Ansprüchen 4 bis 15.

Erfindungsgemäß ist zur Hemmung der Expression eines vorgegebenen Zielgens in einer Zelle vorgesehen, ein 15 bis 49 Basenpaare aufweisendes Oligoribonukleotid mit doppelst rängiger Struktur (dsRNA) in die Zelle einzuführen, wobei ein Strang der dsRNA einen zum Zielgen zumindest abschnittsweise komplementären höchstens 49 aufeinanderfolgende Nukleotidpaare aufweisenden Bereich I aufweist und ein innerhalb der doppelsträngigen Struktur komplementärer Bereich II aus zwei separaten RNA-Einzelsträngen gebildet wird. Das Oligoribonukleotid weist zumindest abschnittsweise eine definierte Nukleotidsequenz auf. Der definierte Abschnitt kann auf den komplementären Bereich I beschränkt sein. Es kann aber auch sein, daß das doppelsträngige Oligoribonukleotid insgesamt eine definierte Nukleotidsequenz aufweist. Die dsRNA kann länger als der zum Zielgen komplementäre Bereich I sein. Der komplementäre Bereich I kann endständig angeordnet oder in die dsRNA eingeschaltet sein. Eine solche dsRNA kann synthetisch bzw. enzymatisch mit gängigen Verfahren hergestellt werden.

Es hat sich überraschenderweise gezeigt, daß bereits bei einer Länge des komplementären Breichs I von höchstens 49 Basenpaaren eine wirksame Hemmung der Expression des Zielgens erreicht werden kann. Entsprechende Oligoribonukleotide können mit geringerem Herstellungsaufwand bereitgestellt werden.

Insbesondere dsRNA mit einer Länge von mehr als 50 Nukleotidpaaren induziert in Säugerzellen und humanen Zellen bestimmte zelluläre Mechanismen, z.B. die dsRNA-abhängige Proteinkinase oder das 2-5A-System. Das führt zum Verschwinden des durch die eine definierte Sequenz aufweisende dsRNA vermittelten Interferenzeffektes. Dadurch wird die Proteinbiosynthese in der Zelle blockiert. Insbesondere dieser Nachteil wird durch die vorliegende Erfindung beseitigt.

Weiterhin ist die Aufnahme von dsRNA mit kurzer Kettenlänge in die Zelle bzw. in den Zellkern gegenüber längerkettigen dsRNAs deutlich erleichtert.

Es hat sich als vorteilhaft erwiesen, daß die dsRNA verpackt in micellare Strukturen, vorzugsweise in Liposomen, vorliegt. Die dsRNA kann gleichfalls in virale natürliche Kapside oder in auf chemischem oder enzymatischem Weg hergestellte künstliche Kapside oder davon abgeleitete Strukturen eingeschlossen sein. - Die vorgenannten Merkmale ermöglichen ein Einschleusen der dsRNA in vorgegebene Zielzellen.

Das zu hemmende Gen wird zweckmäßigerweise in eukaryontischen Zellen exprimiert. Das Zielgen kann aus der folgenden Gruppe ausgewählt sein: Onkogen, Cytokin-Gen, Id-Protein-Gen, Entwicklungsgen, Priongen. Es kann auch in pathogenen Organismen, vorzugsweise in Plasmodien, exprimiert werden. Es kann Bestandteil eines, vorzugsweise humanpathogenen, Virus oder Viroids sein. - Das vorgeschlagene Verfahren ermöglicht die Herstellung von Mitteln zur Therapie genetisch gesteuerter Krankheiten, z.B. Krebs, viraler Erkrankungen oder Morbus Alzheimer.

Das Virus oder Viroid kann auch ein tier- oder planzenpathogenes Virus oder Viroid sein. In diesem Fall erlaubt das erfindungsgemäße Verfahren auch die Bereitstellung von Mitteln zur Behandlung von Tier- oder Pflanzenkrankheiten.

Nach einem weiteren Ausgestaltungsmerkmal ist die dsRNA abschnittsweise doppelsträngig ausgebildet. Die Enden der dsRNA können modifiziert werden, um einem Abbau in der Zelle oder einer Dissoziation in die Einzelstränge entgegenzuwirken. Eine Dissoziation tritt insbesondere bei Verwendung niedriger Konzentrationen oder kurzer Kettenlängen auf. Zur besonders wirksamen Hemmung der Dissoziation kann der durch die Nukleotidpaare bewirkte Zusammenhalt des komplementären Bereichs II durch mindestens eine, vorzugsweise zwei, weitere chemische Verknüpfung/en erhöht werden. - Eine erfindungsgemäße dsRNA, deren Dissoziation vermindert ist, weist eine höhere Stabilität gegen enzymatischen und chemischen Abbau in der Zelle bzw. im Organismus auf.

Die chemische Verknüpfung wird zweckmäßigerweise durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenkoordination gebildet. Sie kann nach einem besonders vorteilhaften Ausgestaltungsmerkmal an mindestens einem, vorzugsweise an beiden, Ende/n des komplementären Bereichs II hergestellt werden.

Es hat sich weiter als vorteilhaft erwiesen, daß die chemische Verknüpfung mittels einer oder mehrerer Verbindungsgruppen gebildet wird, wobei die Verbindungsgruppen vorzugsweise Poly-(oxyphosphinicooxy-1,3-propandiol)- und/oder Polyethylenglycol-Ketten sind. Die chemische Verknüpfung kann auch durch in den komplementären Bereichen II anstelle von Purinen benutzte Purinanaloga gebildet werden. Von Vorteil ist es ferner, daß die chemische Verknüpfung durch in den komplementären Bereichen II eingeführte Azabenzoleinheiten gebildet wird. Sie kann außerdem durch in den komplementären Bereichen II anstelle von Nukleotiden benutzte verzweigte Nukleotidanaloga gebildet werden.

Es hat sich als zweckmäßig erwiesen, daß zur Herstellung der chemischen Verknüpfung mindestens eine der folgenden Gruppen benutzt wird: Methylenblau; bifunktionelle Gruppen, vorzugsweise Bis-(2-chlorethyl)-amin; N-acetyl-N'-(p-glyoxyl-benzoyl)-cystamin; 4-Thiouracil; Psoralen. Ferner kann die chemische Verknüpfung durch an den Enden des doppelsträngigen Bereichs angebrachte Thiophosphoryl-Gruppen gebildet werden. Vorzugsweise wird die chemische Verknüpfung an den Enden des doppelsträngigen Bereichs durch Tripelhelix-Bindungen hergestellt.

Die chemische Verknüpfung kann zweckmäßigerweise durch ultraviolettes Licht induziert werden.

Die Nukleotide der dsRNA können modifiziert sein. Dies wirkt einer Aktivierung einer von doppelsträngiger RNA abhängigen Proteinkinase, PKR, in der Zelle entgegen. Vorteilhafterweise ist mindestens eine 2'-Hydroxylgruppe der Nukleotide der dsRNA in dem komplementären Bereich II durch eine chemische Gruppe, vorzugsweise eine 2'-Amino- oder eine 2'-Methylgruppe, ersetzt. Mindestens ein Nukleotid in mindestens einem Strang des komplementären Bereichs II kann auch ein sogenanntes "locked nucleotide" mit einem, vorzugsweise durch eine 2'-O, 4'-C-Methylenbrücke, chemisch modifizierten Zuckerring sein. Vorteilhafterweise sind mehrere Nukleotide "locked nucleotides".

Nach einer weiteren besonders vorteilhaften Ausgestaltung ist vorgesehen, daß die dsRNA an mindestens ein von einem Virus stammendes, davon abgeleitetes oder ein synthetisch hergestelltes virales Hüllprotein gebunden, damit assoziiert oder davon umgeben wird. Das Hüllprotein kann vom Polyomavirus abgeleitet sein. Es kann das Hüllprotein das Virus-Protein 1 (VP1) und/oder das Virus-Protein 2 (VP2) des Polyomavirus enthalten. Die Verwendung derartiger Hüllproteine ist z.B. aus der DE 196 18 797 A1 bekannt, deren Offenbarungsgehalt hiermit einbezogen wird. - Die vorgenannten Merkmale erleichtert wesentlich das Einführen der dsRNAs in die Zelle.

Vorzugsweise ist bei Bildung eines Kapsids oder kapsidartigen Gebildes aus dem Hüllprotein die eine Seite zum Inneren des Kapsids oder kapsidartigen Gebildes gewandt. Das gebildete Konstrukt ist besonders stabil.

Die dsRNA kann zum primären oder prozessierten RNA-Transkript des Zielgens komplementär sein. - Die Zelle kann eine Vertebratenzelle oder eine menschliche Zelle sein.

Es können mindestens zwei voneinander verschiedene dsRNAs in die Zelle eingeführt werden, wobei ein Strang jeder dsRNA zumindest abschnittsweise komplementär zu jeweils einem von mindestens zwei verschiedenen Zielgenen ist. Dadurch ist es möglich gleichzeitig die Expression mindestens zwei verschiedener Zielgene zu hemmen. Um die Expression einer von doppelsträngiger RNA abhängigen Proteinkinase, PKR, in der Zelle zu unterdrücken, ist eines der Zielgene vorteilhafterweise das PKR-Gen. Dadurch kann die PKR-Aktivität in der Zelle wirksam unterdrückt werden.

Nach Maßgabe der Erfindung ist ferner ein Medikament mit mindestens einem 15 bis 49 Basenpaare aufweisenden Oligoribonukleotid mit doppelsträngiger Struktur (dsRNA) zur Hemmung der Expression eines vorgegebenen Zielgens in Säugerzellen vorgesehen, wobei ein Strang der dsRNA einen zum Zielgen zumindest abschnittsweise komplementären höchstens 49 aufeinanderfolgende Nukleotidpaare aufweisenden Bereich I aufweist, und ein innerhalb der doppelsträngigen Struktur komplementärer Bereich II aus zwei separaten RNA-Einzelsträngen gebildet ist. - Es hat sich überraschend gezeigt, daß eine solche dsRNA sich als Medikament zur Hemmung der Expression eines vorgegebenen Gens in Säugerzellen eignet. Die Hemmung wird im Vergleich zur Verwendung einzelsträngiger Oligoribonukleotide bereits bei Konzentrationen bewirkt, die um mindestens eine Größenordnung niedriger sind. Das erfindungsgemäße Medikament ist hoch wirksam. Es sind geringere Nebenwirkungen zu erwarten. Es hat sich überraschenderweise gezeigt, daß bereits bei einer Länge des komplementären Bereichs I von höchstens 49 Basenpaaren eine effiziente Hemmung der Expression des Zielgens erreicht werden kann. Entsprechende Oligoribonukleotide können mit geringerem Herstellungsaufwand bereitgestellt werden.

Nach weiterer Maßgabe der Erfindung ist eine Verwendung eines 15 bis 49 Basenpaare aufweisenden Oligoribonukleotids mit doppelsträngiger Struktur (dsRNA) zur Herstellung eines Medikaments zur Hemmung der Expression eines vorgegebenen Zielgens in Säugerzellen vorgesehen, wobei ein Strang der dsRNA einen zum Zielgen zumindest abschnittsweise komplementären höchstens 49 aufeinanderfolgende Nukleotidpaare aufweisenden Bereich I aufweist und ein innerhalb der doppelsträngigen Struktur komplementärer Bereich II aus zwei separaten RNA-Einzelsträngen gebildet ist. - Überraschenderweise eignet sich eine solche dsRNA zur Herstellung eines Medikaments zur Hemmung der Expression eines vorgegebenen Gens. Bei einer Verwendung von dsRNA wird die Hemmung im Vergleich zur Verwendung einzelsträngiger Oligoribonukleotide schon bei um eine Größenordnung geringeren Konzentrationen bewirkt. Die erfindungsgemäße Verwendung ermöglicht also die Herstellung besonders wirksamer Medikamente.

Hinsichtlich vorteilhafter Ausgestaltungen des Medikaments und der Verwendung wird auf die Beschreibung der vorangegangenen Merkmale verwiesen.

Nachfolgend werden anhand der Figuren Ausführungsbeispiele der Erfindung näher erläutert. Es zeigen:
- Fig. 1: die schematische Darstellung eines Plasmids für die *in vitro*-Transkription mit T7- und SP6-Polymerase,
- Fig. 2: RNA nach Elektrophorese auf einem 8%igen Polyacrylamidgel und Ethidiumbromidfärbung,
- Fig. 3: eine Darstellung radioaktiver RNA-Transkripte nach Elektrophorese auf einem 8%igen Polyacrylamidgel mit 7 M Harnstoff mittels eines "Instant Imagers" und
- Fig. 4 a - e: Texas-Rot- und YFP-Fluoreszenz in murinen Fibroblasten.

### Ausführungsbeispiel 1:

Die Inhibition der Transkription wurde durch sequenzhomologe dsRNA in einem *in vitro*-Transkriptionssystem mit einem Kernextrakt aus humanen HeLa-Zellen nachgewiesen. Die DNA-Matrize für diesen Versuch war das mittels *Bam*HI linearisierte Plasmid pCMV1200.

### Herstellung der Matrizenplasmide:

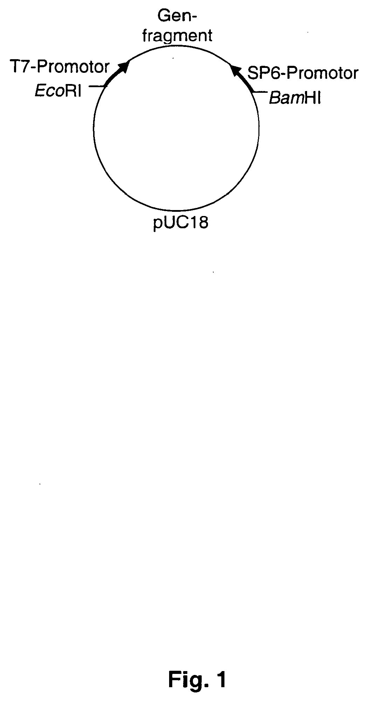

Zur Verwendung bei der enzymatischen Synthese der dsRNA wurde das in Fig. 1 dargestellte Plasmid konstruiert. Dazu wurde zunächst eine Polymerase-Kettenreaktion (PCR) mit der "positive control DNA" des HeLaScribe^{®} Nuclear Extract *in vitro* Transkriptionskits der Firma Promega, Madison, USA als DNA-Matrize durchgeführt. Einer der verwendeten Primer enthielt die Sequenz einer *Eco*RI-Schnittstelle und des T7-RNA-Polymerase-Promotors gemäß Sequenzprotokoll Nr. 1. Der andere Primer enthielt die Sequenz einer *Bam*HI-Schnittstelle und des SP6-RNA-Polymerase-Promotors gemäß Sequenzprotokoll Nr. 2. Darüber hinaus wiesen beide Primer an ihren 3'-Enden identische bzw. komplementäre Bereiche zur DNA-Matrize auf. Die PCR wurde mittels des *"Taq PCR Core Kits"* der Firma Qiagen, Hilden, Deutschland nach Herstellerangaben durchgeführt. In einem Volumen von 100 µl wurden 1,5 mM MgCl₂, je 200 µM dNTP, je 0,5 µM Primer, 2,5 U *Taq*-DNA-Polymerase und etwa 100 ng "positive control DNA" als Matrize in PCR-Puffer eingesetzt. Nach der anfänglichen Denaturierung der Matrizen-DNA durch Erhitzen auf 94°C für 5 Minuten erfolgte die Amplifikation in 30 Zyklen von je 60 Sekunden Denaturierung bei 94°C, 60 Sekunden Annealing bei 5°C unterhalb der berechneten Schmelztemperatur der Primer und 1,5 - 2 Minuten Polymerisation bei 72°C. Nach einer Schlußpolymerisation von 5 Minuten bei 72°C wurden 5 µl des Reaktionsansatzes durch Agarosegelelektrophorese analysiert. Die Länge des so amplifizierten DNA-Fragmentes betrug 400 Basenpaare, wobei 340 Basenpaare der "positive control DNA" entsprachen. Das PCR-Produkt wurde aufgereinigt, mit *Eco*RI und *Bam*HI hydrolysiert und nach erneuter Aufreinigung zur Ligation mit einem ebenfalls durch *Eco*RI und *Bam*HI hydrolysierten pUC18 Vektor eingesetzt. Es erfolgte Transformation von *E*. *coli* XL1-blue. Das erhaltene Plasmid (pCMV5) trägt ein DNA-Fragment, das am 5'-Ende von dem T7- und am 3'-Ende von dem SP6-Promotor flankiert wird. Durch Linearisierung des Plasmids mit *Bam*HI kann es *in vitro* mit der T7-RNA-Polymerase zur *run-off-*Transkription einer 340 Nukleotide langen, in Sequenzprotokoll Nr. 3 dargestellten, einzelsträngigen RNA eingesetzt werden. Wird das Plasmid mit *Eco*RI linearisiert, kann es zur *run-off-*Transkription mit der SP6-RNA-Polymerase eingesetzt werden, wobei der komplementäre Strang entsteht. Entsprechend dem zuvor dargestellten Verfahren wurde auch eine 23 Nukleotide längere RNA synthetisiert. Dazu wurde eine in Sequenzprotokoll Nr. 4 dargestellte DNA über die *Eco*RI und *Bam*HI-Schnittstellen mit dem pUC18 Vektor ligiert.

Als DNA-Matrize für die in vitro-Transkription mit HeLa-Kernextrakt wurde das Plasmid pCMV1200 konstruiert. Dazu wurde ein 1191 bp großes EcoRI/BamHI-Fragment der im HeLaScribe^{®} Nuclear Extract *in vitro* Transkriptionskit enthaltenen Positivkontroll-DNA mittels PCR amplifiziert. Das amplifizierte Fragment umfaßt den 828 bp großen "unmittelbar frühen" CMV-Promotor und ein 363 bp großes transkribierbares DNA-Fragment. Das PCR-Produkt wurde über "T-Überhang"-Ligation mit dem Vektor pGEM-T ligiert. Am 5'-Ende des Fragments ist eine BamHI-Schnittstelle. Das Plasmid wurde durch Hydrolyse mit BamHI linearisiert und als Matrize zur run-off-Transkription eingesetzt.

### in vitro-Transkription der komplementären Einzelstränge:

pCMVS-Plasmid-DNA wurde mit *Eco*RI bzw. *Bam*HI linearisiert. Sie wurde als DNA-Matrize für eine *in vitro*-Transkription der komplementären RNA-Einzelstränge mit SP6- bzw. T7-RNA-Polymerase verwendet. Dazu wurde das "Riboprobe *in vitro* Transcription" System der Firma Promega, Madison, USA eingesetzt. Nach Herstellerangaben wurden 2 µg linearisierte Plasmid-DNA in 100 µl Transkriptionspuffer und 40 U T7- oder SP6-RNA-Polymerase 5 - 6 Stunden bei 37°C inkubiert. Anschließend wurde die DNA-Matrize durch Zugabe von 2,5 µl RNase-freier DNase RQ1 und Inkubation für 30 Minuten bei 37°C abgebaut. Der Transkriptionsansatz wurde mit H₂O auf 300 µl aufgefüllt und durch Phenolextraktion gereinigt. Die RNA wurde durch Zugabe von 150 µl 7 M Ammoniumacatat und 1125 µl Ethanol gefällt und bis zur Hybridisierung bei -65°C aufbewahrt.

### Herstellung der RNA-Doppelstränge:

Zur Hybridisierung wurden 500 µl der in Ethanol aufbewahrten und gefällten einzelsträngigen RNA abzentrifugiert. Das resultierende Pellet wurde getrocknet und in 30 µl PIPES-Puffer, pH 6,4 in Gegenwart von 80 % Formamid, 400 mM NaCl und 1 mM EDTA aufgenommen. Jeweils 15 µl der komplementären Einzel stränge wurden zusammengegeben und für 10 Minuten auf 85°C erhitzt. Anschließend wurden die Ansätze bei 50°C über Nacht inkubiert und auf Raumtemperatur abgekühlt.

Bei der Hybridisierung wurden nur annähernd äquimolare Mengen der beiden Einzelstränge eingesetzt. Dadurch enthielten die dsRNA-Präparationen einzelsträngige RNA (ssRNA) als Kontamination. Um diese ssRNA-Kontaminationen zu entfernen, wurden die Ansätze nach der Hybridisierung mit den einzelstrangspezifischen Ribonukleasen RNase A aus Rinderpankreas und RNase T1 aus *Aspergillus oryzae* behandelt. RNase A ist eine für Pyrimidine spezifische Endoribonuklease. RNase T1 ist eine Endoribonuklease, die bevorzugt auf der 3'-Seite von Guanosinen schneidet. dsRNA ist kein Substrat für diese Ribonukleasen. Für die RNase-Behandlung wurde zu den Ansätzen in 300 µl Tris, pH 7,4, 300 mM NaCl und 5 mM EDTA 1,2 µl RNaseA in einer Konzentration von 10 mg/ml und 2 µl RNaseT1 in einer Konzentration von 290 µg/ml zugegeben. Die Ansätze wurden 1,5 Stunden bei 30°C inkubiert. Danach wurden die RNasen durch Zugabe von 5 µl Proteinase K in einer Konzentration von 20 mg/ml sowie 10 µl 20%iges SDS und Inkubation für 30 Minuten bei 37°C denaturiert. Die dsRNA wurde durch Phenol-Extraktion gereinigt und mit Ethanol gefällt. Um die Vollständigkeit des RNase-Verdaus überprüfen zu können, wurden zwei Kontrollansätze mit ssRNA analog zu den Hybridisierungsansätzen behandelt.

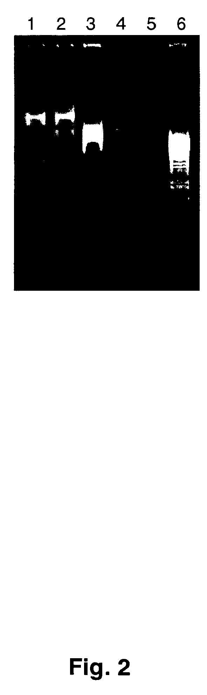

Das getrocknete Pellet wurde in 15 µl TE-Puffer, pH 6,5 aufgenommen und auf einem 8%igen Gel einer nativen Polyacrylamidgelelektrophorese unterzogen. Das Acrylamidgel wurde anschließend in einer Ethidiumbromidlösung gefärbt und in einem Wasserbad gespült. Fig. 2 zeigt die auf einem UV-Transilluminator sichtbar gemachte RNA. Die auf Spur 1 aufgetragene sense- und die auf Spur 2 aufgetragene *antisense*-RNA zeigten unter den gewählten Bedingungen ein anderes Laufverhalten als die auf Spur 3 aufgetragene dsRNA des Hybridisierungsansatzes. Die auf den Spuren 4 bzw. 5 aufgetragene RNase-behandelte *sense-* bzw *antisense*-RNA erzeugte keine sichtbare Bande. Dies zeigt, daß die einzelsträngigen RNAs vollständig abgebaut wurden. Die auf Spur 6 aufgetragene RNase-behandelte dsRNA des Hybridisierungsansatzes ist resistent gegenüber der RNase-Behandlung. Die im nativen Gel im Vergleich zu der auf Spur 3 aufgetragenen dsRNA schneller wandernde Bande resultiert aus dsRNA, die frei von ssRNA ist. Neben der dominierenden Hauptbande treten nach der RNase-Behandlung schwächere, schneller wandernde Banden auf.

*in vitro*-Transkriptions-Test mit menschlichem Zellkernextrakt: Unter Verwendung des HeLaScribe^{®} Nuclear Extract *in vitro* Transkriptionskits der Firma Promega, Madison, USA wurde die Transkriptionseffizienz des oben angegebenen, im Plasmid pCMV1200 enthaltenen, zur "positive control DNA" homologen DNA-Fragments in Gegenwart der sequenzhomologen dsRNA (dsRNA-CMV5) bestimmt. Außerdem wurde der Einfluß der nichtsequenzhomologen, dem "Gelb fluoreszierenden Protein" (YFP)-Gen entsprechenden dsRNA (dsRNA-YFP) untersucht. Diese dsRNA war analog zur sequenzhomologen dsRNA hergestellt worden. Die Sequenz eines Stranges dieser dsRNA ist Sequenzprotokoll Nr. 5 zu entnehmen. Als Matrize für die *run-off*-Transkription diente das Plasmid pCMV1200. Es trägt den "unmittelbar frühen" Promotor des Cytomegalievirus, der von der eukaryotischen RNA-Polymerase II erkannt wird, und ein transkribierbares DNA-Fragment. Die Transkription erfolgte mittels des HeLa-Kernextrakts, der alle notwendigen Proteine für eine Transkription enthält. Durch Zugabe von [α-³²P]rGTP zum Transkriptionsansatz wurde radioaktiv markiertes Transkript erhalten. Das verwendete [α-³²P]rGTP hatte eine spezifische Aktivität von 400 Ci/mmol, 10 mCi/ml. Pro Ansatz wurden 3 mM MgCl₂, je 400 µM rATP, rCTP, rUTP, 16 µM rGTP, 0,4 µM [α-³²P]rGTP und je nach Versuch 1 fmol linearisierte Plasmid-DNA und verschiedene Mengen an dsRNA in Transkriptionspuffer eingesetzt. Jeder Ansatz wurde mit H₂O auf ein Volumen von 8,5 µl aufgefüllt. Die Ansätze wurden vorsichtig gemischt. Zum Starten der Transkription wurden 4 U HeLa-Kernextrakt in einem Volumen von 4 µl zugegeben und für 60 Minuten bei 30°C inkubiert. Die Reaktion wurde durch Zugabe von 87,5 µl auf 30°C erwärmten Stopp-Mix beendet. Zur Entfernung der Proteine wurden die Ansätze mit 100 µl Phenol/Chloroform/Isoamylalkohol (25:24:1, v/v/v), gesättigt mit TE-Puffer, pH 5,0, versetzt und 1 Minute kräftig gemischt. Zur Phasentrennung wurde etwa 1 Minute bei 12000 rpm zentrifugiert und die obere Phase in ein neues Reaktionsgefäß überführt. Zu jedem Ansatz wurden 250 µl Ethanol zugegeben. Die Ansätze wurden gut gemischt und für mindestens 15 Minuten auf Trockeneis/Methanol inkubiert. Zur Präzipitation der RNA wurden die Ansätze 20 Minuten bei 12000 rpm und 4°C zentrifugiert. Der Überstand wurde verworfen. Das Pellet wurde 15 Minuten im Vakuum getrocknet und in 10 µl H₂O resuspendiert. Zu jedem Ansatz wurden 10 µl denaturierender Probenpuffer zugegeben. Die Trennung des freien GTP vom entstandenen Transkript erfolgte mittels denaturierender Polyacrylamid-Gelelektrophorese auf einem 8%igen Gel mit 7 M Harnstoff. Die bei der Transkription mit HeLa-Kernextrakt gebildeten RNA-Transkripte in denaturierendem Probenpuffer wurden für 10 Minuten auf 90°C erhitzt und 10 µl davon sofort in die frisch gespülten Probentaschen aufgetragen. Die Elektrophorese erfolgte bei 40 mA. Die Menge der bei der Transkription gebildeten radioaktiven ssRNA wurde nach der Elektrophorese mit Hilfe eines *Instant Imager* analysiert.

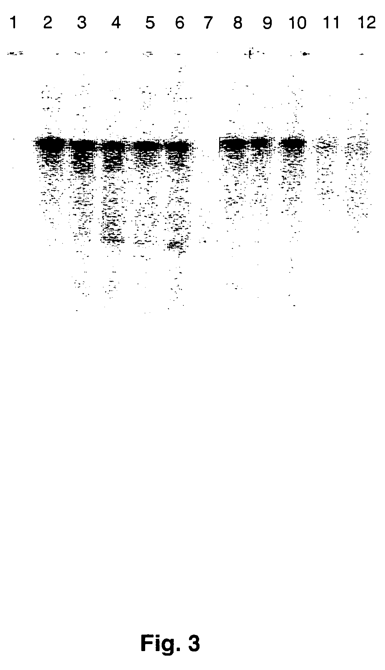

Fig. 3 zeigt die mittels des *Instant Imagers* dargestellte radioaktive RNA aus einem repräsentativen Tests. Es wurden aus folgenden Transkriptionsansätzen gewonne Proben aufgetragen:

| | |
|---|---|
| Spur 1: | ohne Matrizen-DNA, ohne dsRNA; |
| Spur 2: | 50 ng Matrizen-DNA, ohne dsRNA; |
| Spur 3: | 50 ng Matrizen-DNA, 0,5 µg dsRNA-YFP; |
| Spur 4: | 50 ng Matrizen-DNA, 1,5 µg dsRNA-YFP; |
| Spur 5: | 50 ng Matrizen-DNA, 3 µg dsRNA-YFP; |
| Spur 6: | 50 ng Matrizen-DNA, 5 µg dsRNA-YFP; |
| Spur 7: | ohne Matrizen-DNA, 1,5 dsRNA-YFP; |
| Spur 8: | 50 ng Matrizen-DNA, ohne dsRNA; |
| Spur 9: | 50 ng Matrizen-DNA, 0,5 µg dsRNA-CMV5; |
| Spur 10: | 50 ng Matrizen-DNA, 1,5 µg dsRNA-CMV5; |
| Spur 11: | 50 ng Matrizen-DNA, 3 µg dsRNA-CMV5; |
| Spur 12: | 50 ng Matrizen-DNA, 5 µg dsRNA-CMV5; |

Es zeigte sich eine deutliche Verringerung der Menge an Transkript in Gegenwart von sequenzhomologer dsRNA im Vergleich zum Kontrollansatz ohne dsRNA sowie auch zu den Ansätzen mit nicht-sequenzhomologer dsRNA-YFP. Die Positivkontrolle in Spur 2 zeigt, daß bei der *in vitro*-Transkription mit HeLa-Kernextrakt radioaktives Transkript gebildet wurde. Der Ansatz dient zum Vergleich mit den Transkriptionsansätzen, die in Gegenwart von dsRNA inkubiert worden waren. Die Spuren 3 bis 6 zeigen, daß die Zugabe von nicht-sequenzspezifischer dsRNA-YFP keinen Einfluß auf die Menge des gebildeten Transkripts hat. Die Spuren 9 bis 12 zeigen, daß die Zugabe einer zwischen 1,5 und 3 µg liegenden Menge sequenzspezifischer dsRNA-CMV5 zu einer Abnahme der gebildeten Transkript-Menge führt. Um auszuschließen, daß die beobachteten Effekte nicht auf der dsRNA, sondern auf einer möglicherweise bei der Herstellung der dsRNA unabsichtlich mitgeführten Kontamination beruhen, wurde eine weitere Kontrolle durchgeführt. Einzelstrang-RNA wurde wie oben beschrieben transkribiert und anschließend der RNase-Behandlung unterzogen. Mittels nativer Polyacrylamidgelelektrophorese konnte gezeigt werden, daß die ssRNA vollständig abgebaut worden war. Dieser Ansatz wurde wie die Hybridisierungsansätze einer Phenolextraktion und einer Ethanolfällung unterzogen und anschließend in TE-Puffer aufgenommen. Auf diese Weise wurde eine Probe erhalten, die keine RNA enthielt, aber mit den gleichen Enzymen und Puffern behandelt worden war wie die dsRNA. Spur 8 zeigt, daß der Zusatz dieser Probe keinen Einfluß auf die Transkription hatte. Die Abnahme des Transkripts bei Zugabe sequenzspezifischer dsRNA kann deshalb eindeutig der dsRNA selbst zugeschrieben werden. Die Reduzierung der Transkript-Menge eines Gens in Gegenwart von dsRNA bei einem menschlichen Transkriptionssystem zeigt eine Hemmung der Expression des entsprechenden Gens an. Dieser Effekt ist auf einen neuartigen, durch die dsRNA bedingten Mechanismus zurückzuführen.

### Ausführungsbeispiel 2:

Als Testsystem für diese *in vivo*-Experimente diente die murine Fibroblasten-Zellinie NIH3T3, ATCC CRL-1658. Mit Hilfe der Mikroinjektion wurde das YFP-Gen in die Zellkerne eingebracht. Die Expression des YFP wurde unter dem Einfluß gleichzeitig mittransfizierter sequenzhomologer dsRNA untersucht. Diese dsRNA-YFP ist über eine Länge von 315 bp zum 5'-Bereich des YFP-Gens homolog. Die Nukleotidsequenz eines Strangs der dsRNA-YFP ist in Sequenzprotokoll Nr. 5 wiedergegeben. Die Auswertung unter dem Fluoreszenzmikroskop erfolgte 3 Stunden nach Injektion anhand der grün-gelben Fluoreszenz des gebildeten YFP.

Konstruktion des Matrizenplasmids und Herstellung der dsRNA: Als Matrize für die Herstellung der YFP-dsRNA mittels T7- und SP6-*in vitro*-Transkription wurde ein Plasmid nach dem gleichen Prinzip wie im Ausführungsbeispiel 1 beschrieben konstruiert. Das gewünschte Genfragment wurde unter Verwendung des Primers *Eco*_T7_YFP gemäß Sequenzprotokoll Nr. 6 und *Bam*_SP6_YFP gemäß Sequenzprotokoll Nr. 7 mittels PCR amplifiziert und analog zu der obigen Beschreibung zur Herstellung der dsRNA verwendet. Die erhaltene dsRNA-YFP ist identisch mit der in Ausführungsbeispiel 1 als nicht-sequenzspezifische Kontrolle verwendeten dsRNA.

Es wurde eine am 3'-Ende der RNA gemäß Sequenzprotokoll Nr. 8 über eine C18-Linkergruppe chemisch mit dem 5'-Ende der komplementären RNA verknüpfte dsRNA (L-dsRNA) hergestellt. Dazu wurden mit Disulfid-Brücken modifizierte Synthone verwendet. Das 3'-terminale Synthon ist über den 3'-Kohlenstoff mit einer aliphatischen Linker-Gruppe über eine Disulfidbrücke an den festen Träger gebunden. Bei dem zum 3'-terminalen Synthon des einen Oligoribonukleotids komplementären 5'-terminalen Synthon des komplementären Oligoribonukleotids ist die 5'-Tritylschutzgruppe über einen weiteren aliphatischen Linker und eine Disulfidbrücke gebunden. Nach Synthese der beiden Einzelstränge, Entfernen der Schutzgruppen und Hybridisierung der komplementären Oligoribonukleotide gelangen die entstehenden Thiolgruppen in räumliche Nachbarschaft zueinander. Durch Oxidation werden die Einzelstränge über ihre aliphatischen Linker und eine Disulfidbrücke miteinander verknüpft. Anschließend erfolgt Reinigung mit Hilfe der HPLC.

### Vorbereitung der Zellkulturen:

Die Zellen wurden in DMEM mit 4,5 g/l Glucose, 10 % fötalem Rinderserum unter 7,5 % CO₂-Atmosphäre bei 37°C in Kulturschalen inkubiert und vor Erreichen der Konfluenz passagiert. Das Ablösen der Zellen erfolgte mit Trypsin/EDTA. Zur Vorbereitung der Mikroinjektion wurden die Zellen in Petrischalen überführt und bis zu Bildung von Mikrokolonien weiter inkubiert.

### Mikroinjektion:

Die Kulturschalen wurde zur Mikroinjektion für ca. 10 Minuten aus dem Inkubator genommen. Es wurde in ca. 50 Zellkerne pro Ansatz innerhalb eines markierten Bereichs unter Verwendung des Mikroinjektionssystems AIS der Firma Carl Zeiss, Göttingen, Deutschland einzeln injiziert. Anschließend wurden die Zellen weitere drei Stunden inkubiert. Für die Mikroinjektion wurden Borosilikat-Glaskapillaren der Firma Hilgenberg GmbH, Malsfeld, Deutschland mit einem Spitzendurchmesser unter 0,5 µm vorbereitet. Die Mikroinjektion wurde mit einem Mikromanipulator der Firma Narishige Scientific Instrument Lab., Tokyo, Japan durchgeführt. Die Injektionsdauer betrug 0,8 Sekunden, der Druck ca. 100 hPa. Für die Transfektion wurde das Plasmid pCDNA-YFP verwendet, das ein ca. 800 bp großes *Bam*HI/*Eco*RI-Fragment mit dem Gen des YFP im Vektor pcDNA3 enthält. Die in die Zellkerne injizierten Proben enthielten 0,01 µg/µl pCDNA-YFP sowie an Dextran-70000 gekoppeltes Texas-Rot in 14 mM NaCl, 3 mM KCl, 10 mM KPO₄, pH 7,5. Zusätzlich wurden ca. 100 pl RNA mit einer Konzentration von 1 µM, bzw. 375 µM im Fall der L-dsRNA, zugegeben.

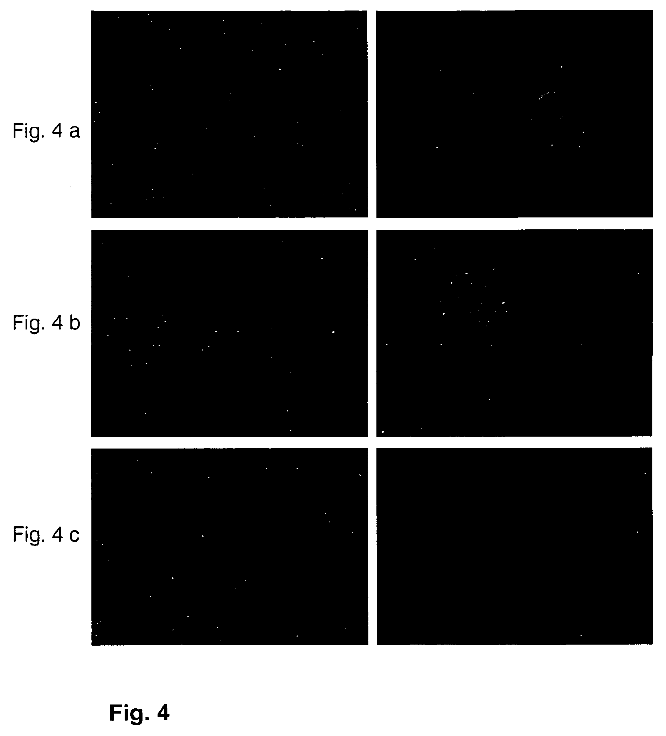

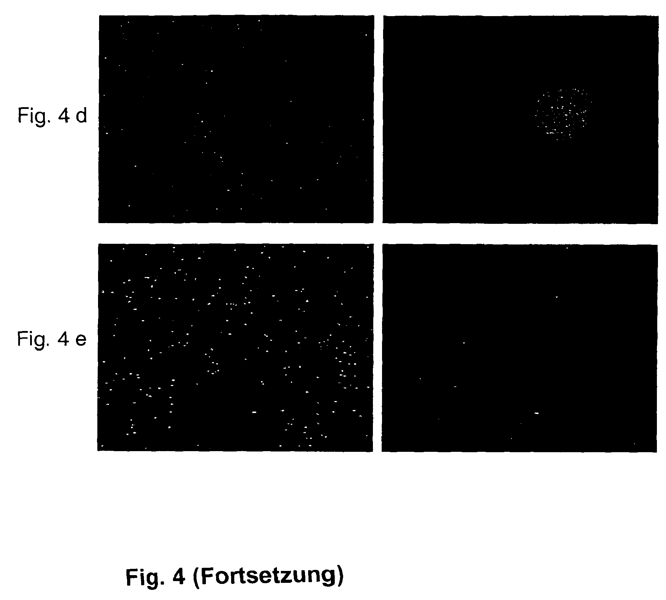

Die Zellen wurden bei Anregung mit Licht der Anregungswellenlänge von Texas-Rot, 568 nm, bzw. von YFP, 488 nm, mittels eines Fluoreszenzmikroskops untersucht. Einzelne Zellen wurden mittels einer digitalen Kamera dokumentiert. Die Figuren 4 a - e zeigen das Ergebnis für NIH3T3-Zellen. Bei den in Fig. 4 a gezeigten Zellen ist sense-YFP-ssRNA, in Fig. 4b *antisense-*YFP-ssRNA, in Fig. 4c dsRNA-YFP, in Fig. 4d keine RNA und in Fig. 4e L-dsRNA injiziert worden.

Das jeweils linke Feld zeigt die Fluoreszenz von Zellen, die mit 568 nm angeregt wurden. Rechts ist die Fluoreszenz derselben Zellen bei Anregung mit 488 nm zu sehen. Die Texas-Rot-Fluoreszenz aller dargestellten Zellen zeigt, daß die Injektionslösung erfolgreich in die Zellkerne appliziert wurde und getroffene Zellen nach drei Stunden noch lebendig waren. Abgestorbene Zellen zeigten keine Texas-Rot-Fluoreszenz mehr.

Die jeweils rechten Felder der Figuren 4 a und 4 b zeigen, daß die Expression des YFP bei Injektion der einzelsträngigen RNA in die Zellkerne nicht sichtbar inhibiert wurde. Das rechte Feld der Fig. 4c zeigt Zellen, deren YFP-Fluoreszenz nach Injektion von dsRNA-YFP nicht mehr nachweisbar war. Fig. 4 d zeigt als Kontrolle Zellen, in die keine RNA injiziert worden war. Die in Fig. 4e dargestellte Zelle zeigt durch die Injektion der L-dsRNA, die zum YFP-Gen sequenzhomologe Bereiche aufweist, eine nicht mehr nachweisbare YFP-Fluoreszenz. Dieses Ergebnis belegt, daß auch kürzere dsRNAs zur spezifischen Inhibition der Genexpression bei Säugern verwendet werden können, wenn die Doppelstränge durch chemische Verknüpfung der Einzelstränge stabilisiert werden.

### Literatur:

Asanuma, H., Ito, T., Yoshida, T., Liang, X. & Komiyama, M. (1999). Photoregulation der Bildung und Dissoziation eines DNA-Duplexes durch cis-trans-Isomerisierung einer Azobenzoleinheit. Angew. Chem. 111, 2547-2549.
Azhayeva, E., Azhayev, A., Auriola, S., Tengvall, U., Urtti, A. & Lönnberg, H. (1997). Inhibitory properties of double helix forming circular oligonucleotides. Nucl. Acids Res. 25, 4954-4961.
Castelli, J., Wood, K.A. & Youle, R.J. (1998). The 2-5A system in viral infection and apoptosis. Biomed. Pharmacother. 52, 386-390.
Dolinnaya, N.G., Blumenfeld, M., Merenkova, I., Oretskaya, T.S., Krynetskaya, N.F., Ivanovskaya, M.G., Vasseur, M. & Shabarova, Z.A. (1993). Oligonucleotide circularization by template-directed chemical ligation. Nucl. Acids Res. 21, 5403-5407.
Expert-Bezancon, A., Milet, M. & Carbon, P. (1983). Precise localization of several covalent RNA-RNA cross-link in Escherichia coli 16S RNA. Eur. J. Biochem. 136, 267-274.
Fire, A., Xu, S., Montgomery, M.K., Kostas, S.A., Driver, S.E. & Mello, C.C. (1998). Potent and specific genetic interference by double-stranded RNA in Caenorhabditis elegans. Nature 391, 806-811.
Gao, H., Yang, M., Patel, R. & Cook, A.F. (1995). Circulaization of oligonucleotides by disulfide bridge formation. Nucl. Acids Res. 23, 2025-2029.
Gryaznov, S.M. & Letsinger, R.L. (1993). Template controlled coupling and recombination of oligonucleotide blocks containing thiophosphoryl groups. Nucl. Acids Res. 21, 1403-1408.
Kaufman, R.J. (1999). Double-stranded RNA-activated protein kinase mediates virus-induced apoptosis: A new role for an old actor. Proc. Natl. Acad. Sci. USA 96, 11693-11695.
Lipson, S.E. & Hearst, J.E. (1988). Psoralen cross-linking of ribosomal RNA. In Methods in Enzymology Anonymous pp. 330-341.
Liu, Z.R., Sargueil, B. & Smith, C.W. (1998). Detection of a novel ATP-dependent cross-linked protein at the 5' splice site-U1 small nuclear RNA duplex by methylene bluemediated photo-cross-linking. Mol. Cell. Biol. 18, 6910-6920.
Micura, R. (1999). Cyclic oligoribonucleotides (RNA) by solidphase synthesis. Chem. Eur. J. 5, 2077-2082.
Skripkin, E., Isel, C., Marquet, R., Ehresmann, B. & Ehresmann, C. (1996). Psoralen crosslinking between human immunodeficiency virus type 1 RNA and primer tRNA3Lys. Nucl. Acids Res. 24, 509-514.
Wang, S. & Kool, E.T. (1994). Circular RNA oligonucleotides. Synthesis, nucleic acid binding properties, and a comparison with circular DNAs. Nucl. Acids Res. 22, 2326-2333.
Wang, Z. & Rana, T.M. (1996). RNA conformation in the Tat-TAR complex determined by site-specific photo-cross-linking. Biochem. 35, 6491-6499.
Watkins, K.P. & Agabian, N. (1991). In vivo UV cross-linking of U snRNAs that paticipate in trypanosome transsplicing. Genes & Development 5, 1859-1869.
Wengel, J. (1999). Synthesis of 3'-C- and 4'-C-branched oligodeoxynucleotides and the development of locked nucleic acid (LNA). Acc. Chem. Res. 32, 301-310.
Zwieb, C., Ross, A., Rinke, J., Meinke, M. & Brimacombe, R. (1978). Evidence for RNA-RNA cross-link formation in Escherichia coli ribosomes. Nucl. Acids Res. 5, 2705-2720.

Die Erfindung umfasst ferner die folgenden Ausführungsformen:
1. Oligoribonukleotid mit doppelsträngiger Struktur (dsRNA) zur Hemmung der Expression eines vorgegebenen Zielgens in Säugerzellen, wobei die dsRNA 15 bis 49 Basenpaare aufweist und ein Strang der dsRNA einen zum Zielgen zumindest abschnittsweise komplementären, höchstens 49 aufeinanderfolgende Nukleotidpaare aufweisenden Bereich I aufweist und ein innerhalb der doppelsträngigen Struktur komplementärer Bereich II aus zwei separaten RNA-Einzelsträngen gebildet ist.
2. DsRNA nach Ausführungsform 1, wobei die dsRNA 15 bis 21 Basenpaare aufweist.
3. DsRNA nach einer der Ausführungsformen 1 oder 2, wobei die dsRNA 21 bis 49 Basenpaare aufweist.
4. DsRNA nach einer der vorhergehenden Ausführungsformen, wobei die dsRNA 21 Basenpaare aufweist.
5. DsRNA nach einer der vorhergehenden Ausführungsformen, wobei das Zielgen aus der folgenden Gruppe ausgewählt ist: Onkogen, Cytokin-Gen, Id-Protein-Gen, Entwicklungsgen, PKR-Gen, Priongen.
6. DsRNA nach einer der vorhergehenden Ausführungsformen, wobei der Bereich I vollständig komplementär zum Zielgen ist.
7. DsRNA nach einer der vorhergehenden Ausführungsformen, wobei die dsRNA verpackt in micellare Strukturen, vorzugsweise in Liposomen, vorliegt.
8. DsRNA nach einer der vorhergehenden Ausführungsformen, wobei die dsRNA in virale natürliche Kapside oder in auf chemischem oder enzymatischem Weg hergestellte künstliche Kapside oder davon abgeleitete Strukturen eingeschlossen ist.
9. DsRNA nach einer der vorhergehenden Ausführungsformen, wobei das Zielgen Bestandteil eines Virus oder Viroids ist.
10. DsRNA nach Ausführungsformen 9, wobei das Virus ein humanpathogenes Virus oder Viroid ist.
11. DsRNA nach Ausführungsformen 9, wobei das Virus oder Viroid ein tierpathogenes Virus oder Viroid ist.
12. DsRNA nach einer der vorhergehenden Ausführungsformen, wobei die dsRNA abschnittsweise doppelsträngig ausgebildet ist.
13. DsRNA nach einer der vorhergehenden Ausführungsformen, wobei die Enden der dsRNA modifiziert sind, um einem Abbau in den Säugerzellen oder einer Dissoziation in die Einzelstränge entgegenzuwirken.
14. DsRNA nach einer der vorhergehenden Ausführungsformen, wobei der durch die Nukleotidpaare bewirkte Zusammenhalt des komplementären Bereichs II durch mindestens eine, vorzugsweise zwei, weitere chemische Verknüpfung/en erhöht ist.
15. DsRNA nach einer der vorhergehenden Ausführungsformen, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenko-ordination gebildet ist.
16. DsRNA nach einer der vorhergehenden Ausführungsformen, wobei die chemische Verknüpfung an mindestens einem, vorzugsweise an beiden, Enden des komplementären Bereichs II hergestellt ist.
17. DsRNA nach einer der vorhergehenden Ausführungsformen, wobei die chemische Verknüpfung mittels einer oder mehrerer Verbindungsgruppen gebildet ist, wobei die Verbindungsgruppen vorzugsweise Poly-(oxyphosphinicooxy-1,3-propandiol)- und/oder Polyethylenglycol-Ketten sind.
18. DsRNA nach einer der vorhergehenden Ausführungsformen, wobei die chemische Verknüpfung durch in den komplementären Bereichen II anstelle von Purinen benutzte Purinanaloga gebildet ist.
19. DsRNA nach einer der vorhergehenden Ausführungsformen, wobei die chemische Verknüpfung durch in die komplementären Bereiche II eingeschaltete Azabenzoleinheiten gebildet ist.
20. DsRNA nach einer der vorhergehenden Ausführungsformen, wobei die chemische Verknüpfung durch in den komplementären Bereichen II anstelle von Nukleotiden benutzte verzweigte Nukleotidanaloga gebildet ist.
21. DsRNA nach einer der vorhergehenden Ausführungsformen, wobei zur Herstellung der chemischen Verknüpfung mindestenes eine der folgenden Gruppen benutzt wird: Methylenblau; bifunktionelle Gruppen, vorzugsweise Bis-(2-chlorethyl)-amin; N-acetyl-N'-(p-glyoxyl-benzoyl)-cystamin; 4-Thiouracil; Psoralen.
22. DsRNA nach einer der vorhergehenden Ausführungsformen, wobei die chemische Verknüpfung durch an den Enden des doppelsträngigen Bereichs vorgesehene Thiophosphoryl-Gruppen gebildet ist.
23. DsRNA nach einer der vorhergehenden Ausführungsformen, wobei die chemische Verknüpfung an den Enden des doppelsträngigen Bereichs vorgesehene Tripelhelix-Bindungen sind.
24. DsRNA nach einer der vorhergehenden Ausführungsformen, wobei die Nukleotide der dsRNA modifiziert sind.
25. DsRNA nach einer der vorhergehenden Ausführungsformen, wobei mindestens eine 2'-Hydroxylgruppe der Nukleotide der dsRNA in dem komplementären Bereich II durch eine chemische Gruppe, vorzugsweise eine 2'-Amino- oder eine 2'-Methylgruppe, ersetzt ist.
26. DsRNA nach einer der vorhergehenden Ausführungsformen, wobei mindestens ein Nukleotid in mindestens einem Strang des komplementären Bereichs II ein "locked nucleotide" mit einem, vorzugsweise durch eine 2'-O, 4'-C-Methylenbrücke, chemisch modifizierten Zuckerring ist.
27. DsRNA nach einer der vorhergehenden Ausführungsformen, wobei die dsRNA an mindestens ein von einem Virus stammendes, davon abgeleitetes oder ein synthetisch hergestelltes virales Hüllprotein gebunden, damit assoziiert oder davon umgeben ist.
28. DsRNA nach einer der vorhergehenden Ausführungsformen, wobei das Hüllprotein vom Polyomavirus abgeleitet ist.
29. DsRNA nach einer der vorhergehenden Ausführungsformen, wobei das Hüllprotein das Virus-Protein 1 (VP1) und/oder das Virus-Protein 2 (VP2) des Polyomavirus enthält.
30. DsRNA nach einer der vorhergehenden Ausführungsformen, wobei bei Bildung eines Kapsids oder kapsidartigen Gebildes aus dem Hüllprotein die eine Seite zum Inneren des Kapsids oder kapsidartigen Gebildes gewandt ist.
31. DsRNA nach einer der vorhergehenden Ausführungsformen, wobei die dsRNA zum primären oder prozessierten RNA-Transkript des Zielgens komplementär ist.
32. DsRNA nach einer der vorhergehenden Ausführungsformen, wobei die Säugerzellen menschliche Zellen sind.
33. Oligoribonukleotid mit doppelsträngiger Struktur (dsRNA) zur Hemmung der Expression eines vorgegebenen Zielgens in Säugerzellen, wobei die dsRNA 15 bis 49 Basenpaare aufweist und ein Strang der dsRNA einen zum Zielgen komplementären, höchstens 49 aufeinanderfolgende Nukleotidpaare aufweisenden Bereich I aufweist und ein innerhalb der doppelsträngigen Struktur komplementärer Bereich II aus zwei separaten RNA-Einzelsträngen gebildet ist, wobei das Oligoribonukleotid insgesamt eine durch den Bereich I definierte Nukleotidsequenz aufweist, wobei der Bereich I dieselbe Länge wie die dsRNA aufweist.

## Patentansprüche

1. Zwei voneinander verschiedene 15 bis 49 Basenpaare aufweisende Oligoribonukleotide mit doppelsträngiger Struktur (dsRNAs) zur Hemmung der Expression zweier vorgegebener Zielgene in Säugerzellen, wobei ein Strang jeder dsRNA einen höchstens 49 aufeinanderfolgende Nukleotidpaare aufweisenden Bereich I aufweist, der zumindest abschnittsweise komplementär zu jeweils einem von den zwei verschiedenen Zielgenen ist, und ein innerhalb der doppelsträngigen Struktur komplementärer Bereich II jeweils aus zwei separaten RNA Einzelsträngen gebildet wird.

2. Medikament mit zwei voneinander verschiedenen 15 bis 49 Basenpaare aufweisenden Oligoribonukleotiden mit doppelsträngiger Struktur (dsRNAs) zur Hemmung der Expression zweier vorgegebener Zielgene in Säugerzellen, wobei ein Strang jeder dsRNA einen höchstens 49 aufeinanderfolgende Nukleotidpaare aufweisenden Bereich I aufweist, der zumindest abschnittsweise komplementär zu jeweils einem von den zwei verschiedenen Zielgenen ist, und ein innerhalb der doppelsträngigen Struktur komplementärer Bereich II jeweils aus zwei separaten RNA Einzelsträngen gebildet wird.

3. DsRNAs nach Anspruch 1 oder Medikament nach Anspruch 2, wobei die Nukleotide jeder dsRNA modifiziert sind.

4. DsRNAs oder Medikament nach einem der vorhergehenden Ansprüche, wobei mindestens eine 2'-Hydroxylgruppe der Nukleotide jeder dsRNA in dem komplementären Bereich II durch eine chemische Gruppe ersetzt ist.

5. DsRNAs oder Medikament nach Anspruch 4, wobei die chemische Gruppe eine 2'-Amino- oder eine 2'-Methylgruppe ist.

6. DsRNAs oder Medikament nach einem der vorhergehenden Ansprüche, wobei mindestens ein Nukleotid in mindestens einem Strang des komplementären Bereichs II in jeder dsRNA ein "locked nucleotide" mit einem, vorzugsweise durch eine 2'-O, 4'-C-Methylenbrücke, chemisch modifizierten Zuckerring ist.

7. DsRNAs oder Medikament nach einem der vorhergehenden Ansprüche, wobei die Zielgene Bestandteil eines Virus sind.

8. DsRNAs oder Medikament nach Anspruch 1 bis 6, wobei das Zielgen aus der folgenden Gruppe ausgewählt ist: Onkogen, Cytokin-Gen, Id-Protein-Gen, Entwicklungsgen, PKR-Gen, Priongen.

9. DsRNAs oder Medikament nach einem der vorhergehenden Ansprüche, wobei die dsRNAs verpackt in micellare Strukturen vorliegen.

10. DsRNAs oder Medikament nach Anspruch 9, wobei die micellare Struktur ein Liposom ist.

11. DsRNAs oder Medikament nach einem der vorhergehenden Ansprüche, wobei die Enden jeder dsRNA modifiziert sind, um einem Abbau in den Säugerzellen oder einer Dissoziation in die Einzelstränge entgegenzuwirken.

12. DsRNAs oder Medikament nach einem der vorhergehenden Ansprüche, wobei eines der Zielgene das PKR-Gen ist.
